# NEUE EUROPÄISCHE PATENTSCHRIFT

(11) **EP 1 807 031 B2**
(45) Veröffentlichungstag und Bekanntmachung des Hinweises auf die Entscheidung über den Einspruch: **30.05.2018**
(45) Hinweis auf die Patenterteilung: 28.07.2010
(21) Anmeldenummer: 05801418.4
(22) Anmeldetag: 02.11.2005
(51) Int. Cl.: A61F 13/00

(54) **VORRICHTUNG ZUR WUNDBEHANDLUNG UNTER EINSATZ VON UNTERDRUCK**
DEVICE FOR THE TREATMENT OF WOUNDS USING A VACUUM
DISPOSITIF POUR TRAITER UNE PLAIE EN APPLIQUANT UNE DEPRESSION

(30) Priorität: 02.11.2004 DE 202004017052 U
(43) Veröffentlichungstag der Anmeldung: 18.07.2007
(73) Patentinhaber: BSN medical GmbH, 20253 Hamburg (DE)
(72) Erfinder: Riesinger, Birgit, 48149 Münster (DE)
(74) Vertreter: Jostarndt Patentanwalts-AG
(86) Internationale Anmeldenummer: PCT/EP2005/011692
(87) Internationale Veröffentlichungsnummer: WO 2006/048240

(56) Entgegenhaltungen:
- EP-A- 1 177 781
- EP-A2- 1 129 734
- WO-A-01/10363
- WO-A1-01/89431
- WO-A1-96/05873
- WO-A1-99/01173
- WO-A1-03/094813
- WO-A1-2005/123170
- WO-A1-2006/048240
- DE-A1- 10 059 439
- DE-A1- 10 059 439
- DE-A1- 19 517 699
- US-A- 5 549 584
- US-A- 5 549 584
- US-A- 5 636 643
- US-A- 5 636 643
- US-A1- 2002 065 494
- US-A1- 2004 054 338

## Beschreibung

Die Erfindung betrifft eine Vorrichtung zur Wundbehandlung des menschlichen oder tierischen Körpers unter Einsatz von Unterdruck, aufweisend:
- ein gasdichtes Wundabdeckungselement, das im am Körper des Patienten angelegten Zustand einen zwischen der jeweiligen Wunde und dem Wundabdeckungselement verbleibenden Wundraum bildet,
- wenigstens eine Anschlußstelle, die mit dem Wundraum in Kontakt steht und über welche die im Wundraum befindliche Luft evakuiert werden kann,
- wenigstens einen flächenhaften Absorptionskörper, der im Wundraum unterhalb des Wundabdeckungselementes anzuordnen ist.

Aus der US 2004/0030304 A1 ist eine Vorrichtung zur Vakuumtherapie von Wunden bekannt, die einen umhüllten, schaumstoffartigen Absorptionskörper aufweist, dessen Hülle etwa 3 bis 6 mm große Perforationen hat. An dem Absorptionskörper sind ebenfalls Perforationen von derselben Größe eingebracht. Ferner ist oberhalb des umhüllten Absorptionskörpers eine zusätzliche flüssigkeitsdurchlässige Schaumstoff-Einlage angeordnet. Die Wirkung der Vorrichtung beruht auf dem an sich bekannten Prinzip der Wunddrainage, bei der die Absaugung des Wundexsudats durch ein den Unterdruck erzeugendes Mittel erfolgt. Die Absaugung auf dem atmosphärischen Wege ist eben durch die entsprechend großen Poren unterstützt.

Aus der DE 195 17 699 ist eine Vorrichtung zur Vakuumversiegelung einer Wunde bekannt, die eine Abdeckfolie zum flächigen Überdecken und luftdichten Abschließen der Wunde aufweist, so dass unterhalb der Abdeckfolie im Bereich der Wunde ein Raum entsteht, in den ein Drainageschlauch und eine Schaumstoff-Einlage einsetzbar sind.

In der älteren Patentschrift DE 29 53 373 C2 ist ebenfalls eine Vorrichtung zur Wundbehandlung unter Einsatz von Unterdruck gezeigt, bestehend aus einem Wundabdeckungselement, einer darunter liegenden Schaumstoff-Einlage und wenigstens einer mit den Poren der Schaumstoff-Einlage kommunizierten Schlauchleitung.

Der US 66 85 681 B2 ist eine weitere Vorrichtung zur Wundbehandlung unter Einsatz von Unterdruck zu entnehmen, bei der auf die Wundoberfläche ein elastisches Druckverteilungselement gelegt ist, an das wiederum ein Saugrohr angeschlossen ist. Das Saugrohr ist zwischen dem Wundrand und einem darauf angeklebten Wundabdekkungselement geführt. Der durch das Wundabdeckungselement und das Druckverteilungselement begrenzte Wundraum ist mit Zellstoff gefüllt.

Die GB 6 92 578 zeigt ein Operationstuch, dessen mittlere Öffnung an ihrem Rand eine umlaufende polymere Klebeschicht aufweist, mit der das Operationstuch an der Haut des Patienten befestigt werden kann.

Die US 55 49 584 zeigt eine Vorrichtung zur Vakuumtherapie, welche aus einer Wundabdeckung, einer Membranpumpe und einem der Membranpumpe nachgeschalteten, beutelartigen Kollektor besteht. Unterhalb der Wundabdeckung ist ein Pad oder eine lose Schüttung von flüssigkeitsabsorbierenden Fasern angeordnet, die auf einer perforierten Schicht aufliegt, welche wiederum von einer weiteren, adhäsiven Schicht unterlegt ist. Die Fasern sind noch durch eine flüssigkeitspermeable, mehrere Fenster aufweisende, obere Schicht bedeckt, wobei die Fenster jeweils von einem ebenso permeablen Materialabschnitt unterlegt sind. Der so konstruierte Saugkopf scheint kompliziert und teuer in Herstellung zu sein.

Aufgabe der Erfindung ist, eine kostengünstigere Vorrichtung zur Vakuumtherapie von Wunden zu konzipieren, deren Aufbau vereinfacht ist.

Diese Aufgabe ist durch eine Vorrichtung der eingangs genannten Art dadurch gelöst, bei der
- der Absorptionskörper wenigstens eine in eine Hülle eingefasste Lage eines mit superabsorbierenden Partikeln durchsetzten Textilabschnittes ist, wobei die Hülle flüssigkeitsdurchlässig ist und Poren aufweist, deren Größe die der superabsorbierenden Partikeln im Wesentlichen nicht überschreitet,
- der in den Wundraum einzulegende Absorptionskörper ein Anfangsvolumen aufweist, das sich im Laufe des Absorptionsprozesses vergrößert und ein Endvolumen annimmt, so dass die absorbierten Wundsekrete - bedingt durch die Porengröße der Hülle - innerhalb des Absorptionskörpers und damit unterhalb des Wundabdeckungselementes bis zur Entfernung des Absorptionskörpers aus dem Wundraum verbleiben,
- die Lage in Draufsicht auf ihre Flachseite eine Flächenausdehnung hat, die um 3% bis 90% kleiner ist als die der flachgelegten Hülle.

Der Textilabschnitt des Absorptionskörpers besteht vorzugsweise aus Zellstoffasern.

Die Vorrichtung kann ein flüssigkeitsdurchlässiges, schleimhautfreundliches Schutzelement beinhalten, das sich auf einer dem Wundabdeckungselement gegenüber liegenden Seite des Absorptionskörpers erstreckt. Das Schutzelement ist flächenmäßig etwa dem umhüllten Absorptionskörper gleich, vorzugsweise etwas größer. Das schleimhautfreundliches Schutzelement kann in seiner einfachsten Ausführungsform folienartig sein.

Es kann auch aus weichem, offenzelligem Schaumstoff oder einem sehr lockeren Vliesgebilde hergestellt sein. Schließlich kann das Schutzelement eine unterhalb des Absorptionskörpers liegende, lose Schüttung aus Vlies- oder Schaumstoffstücken sein, die nach dem beendeten Absorptionsvorgang aus der Wunde beispielsweise mit einer Pinzette entfernt werden können. Darüber hinaus erfüllt ein voluminöses Gebilde nicht nur die die Schleimhaut schützende Funktion, sondern auch die Funktion eines Absorbers. Der offenzellige Schaumstoff oder das Vliesgebilde können mehrfach größere Poren als die der Hülle haben, so dass das die größeren Partikeln von Wundexsudat aufgenommen werden können.

Das flüssigkeitsundurchlässige Wundabdeckungselement besteht vorzugsweise aus folienartigem Material, das derart steif ist, dass unter normalen Bedingungen, d. h. im nicht benutzten und im am Körper des Patienten angelegten Zustand nicht schrumpft.

Ferner ist es möglich, das flache, folienartige Wundabdeckungselement durch eine schalenförmige, ebenfalls folienartige und vorzugsweise transparente Abdeckung zu ersetzen, die mit einer flachen Umrandung zum Ankleben an die Haut versehen ist. Eine schalen- bzw. glockenförmige Abdeckung kann kostengünstig im Tiefziehverfahren hergestellt werden.

Das Wundabdeckungselement kann an wenigstens einem Teil seiner Fläche transparent sein, damit der Zustand der Wundheilung beobachtet werden kann.

Das Wundabdeckungselement kann auf seiner Außenseite bedruckbar sein.

Der Absorptionskörper kann mit dem Wundabdeckungselement wenigstens punktweise - unter Belassung einer freien Peripherie am Wundabdeckungselement - verklebt sein.

Unter dem Begriff "Anschlußstelle" soll im wesentlichen eine am Wundabdeckungselement eingearbeitete Auslaßöffnung verstanden werden, an die eine Schlauchleitung und/oder ein Druckmanometer, Vakuumpumpe, Vakuumflasche und dgl. anschließbar ist oder in die ein Ventil eingebaut werden kann. Allerdings kann die Anschlußstelle durch ein flüssigkeits- und gasundurchlässiges Elastomerstück ersetzt werden, welches durch eine Nadel, beispielsweise Spritznadel durchstochen werden kann und das sich nach dem Herausziehen der Nadel zur ursprünglichen Form zusammenzieht.

Als Ventil kann ein Einwegrückschlagventil, beispielsweise eine aus dem Bereich der Luftmatratzen oder Schwimmflügel bekannte Ventileinrichtung oder ein aus der Medizintechnik bekanntes Mikroventil zum Einsatz kommen.

Der Unterdruck im Raum unterhalb des Wundabdeckungselementes kann - bei am Körper des Patienten angeklebter Vorrichtung - manuell oder mechanisch bzw. elektrisch erzeugt werden. Eine einfachste manuelle Vakuumerzeugung kann mithilfe einer Injektionsspritze, einer sogenannten Scheerengriff-Vakuumpumpe oder eines bekannten, per Hand zusammendrückbaren Gummibalgs ("Ballpumpe") erfolgen. Zur elektrischen Vakuumerzeugung eignen sich unterschiedliche, marktübliche Vakuumpumpen, die z. B. mit Schlauch und Druckregulierer lieferbar sind.

Bei den länger dauernden Wundheilungsprozessen ist es erforderlich, den Unterdruck regelmäßig zu kontrollieren und aufrechtzuerhalten. Diese Aufgabe kann dadurch gelöst werden, dass mit einfachstem Mittel, wie Injektionsspritze, die ansammelten Gase nach Bedarf aus dem Raum wie bei der Blutentnahme entzogen werden, indem die Spritznadel ins Gummiventil oder in den Schlauch hineingesteckt wird. Die Größe des Unterdrucks kann mithilfe eines an die Auslaßöffnung angeschlossenen Druckmanometers oder eingebauten Vakuum-Indikators ermittelt werden. Selbstverständlich können Vakuumpumpen eingesetzt werden.

Die Vorrichtung kann in Draufsicht auf ihre Flachseite polygonal, oval oder kreisförmig sein.

Die Hülle besteht aus einem flüssigkeitsdurchlässigen, schleimhautverträglichen Natur- oder Kunststoff, an dem die Wundsekrete nicht oder kaum haften. Dies ermöglicht den Transport von flüssigen Wundsekreten in das Absorptionsmaterial. Die Wundsekrete treten durch die Hülle hindurch und werden durch das mit Superabsorbentien angereichertes Absorptionsmaterial aufgesaugt.

Sowohl die Hülle, als auch das innerhalb der Hülle liegende Absorptionsmaterial kann mit einem geruchshemmenden und/oder -neutralisierenden bzw. -maskierenden Zusatz versehen sein, beispielweise mit Aktivkohlefilter.

Von großem Vorteil ist, dass bei Gasevakuierung keine Wundsekretpartikeln mitgerissen werden. Diese verbleiben solange innerhalb der Hülle des Absorptionskörpers bis die ganze Vorrichtung aus dem Körper des Patienten entfernt und entsorgt bzw. der aufgequollene Absorptionskörper ausgewechselt wird.

Vorteilhaft ist, an der Hülle des Absorptionskörpers einen peripheren Überstand an Hüllenmaterial zu hinterlassen, damit eine schmerzhafte Berührung der relativ harten Naht mit der Wundoberfläche begrenzt oder gar vermieden werden kann. Hierbei wird als Überstand das Hüllenmaterial zwischen der Naht und dem äußersten Umfang der Hülle verstanden.

Unterhalb des Wundabdeckungselementes kann ein Druckverteiler angeordnet sein, welcher zwischen dem Wundabdeckungselement und dem umhüllten Absorptionskörper liegt. Der Druckverteiler kann ein Formstück aus gasdurchlässigem Schaumstoff sein, bei dem mehrere Luftpfade für einen gleichmäßig verteilten Luftdurchfluss vorhanden sind.

Der Druckverteiler kann auch wenigstens teilweise durch ein schlaufen- oder mäanderartig verlaufendes Endstück des durch die Anschlussstelle geführten Saugrohrs gebildet sein. Das Endstück des Saugrohrs kann in ein vorzugsweise flaches, mit dem Absorptionskörper zu kontaktierendes Schaumstoffstück eingebettet sein.

Insgesamt ist eine Vorrichtung zur Wundbehandlung unter Einsatz von Unterdruck geschaffen, bei der die Wundsekrete durch das mit Superabsorber durchsetze Vlies des Absorptionskörpers angesaugt werden und innerhalb der den Absorptionskörper umgebenden Hülle verbleiben, ohne aus der Hülle in den abgedeckten Raum zurück zu gelangen, wobei mit zunehmender Absorption die Querschnittsfläche des Absorptionskörpers sich stark, mehrfach vergrößert und an eine Kreisform annähert, und wobei beim Aufrechterhalten des Unterdrucks mithilfe einer an die Vorrichtung anschließbaren Injektionsspritze oder Vakuumpumpe, d. h. bei Gasevakuierung kaum Wundsekrete mitgerissen werden. Darüber hinaus ist es möglich, auf einen zusätzlichen Sammelbehälter und Drainageschlauch zu verzichten. Die Vorrichtung ist für den Patienten komfortabler in Gebrauch.

Ausführungsbeispiele der Erfindung sind anhand der Zeichnung näher erläutert. Die Figuren zeigen:
- Figuren 1a bis 1d: eine an die Haut des Patienten angeklebte Vorrichtung, in einem schematischen Schnitt;
- Fig. 2: die Vorrichtung gemäß der Fig. 1 in einer Explosionsdarstellung;
- Fig. 3: die Vorrichtung gemäß der Fig. 1 in Drauf- sicht auf das Wundabdeckungselement;
- Fig. 4: eine runde Vorrichtung zur Wundbehandlung in Draufsicht,
- Fig. 5: Schnitt A-A- gemäß der Fig. 1d;
- Fig. 6: eine andere Ausführungsform der Vorrich- tung, in einem schematischen Schnitt; ;
- Fig. 7: die Vorrichtung gemäß der Fig. 1, jedoch mit zwei Anschlußstellen, ebenfalls in ei- nem schematischen Schnitt;
- Fig. 8: die Vorrichtung gemäß der Fig. 1 in Drauf- sicht auf ihre Rückseite;
- Fig. 9: eine weitere Ausführungsform der Vorrich- tung, mit einem Druckverteiler, in einer schematischen Draufsicht auf die Flachsei- te der Hülle; und
- Fig. 10: einen Schnitt B-B gemäß der Fig. 9.

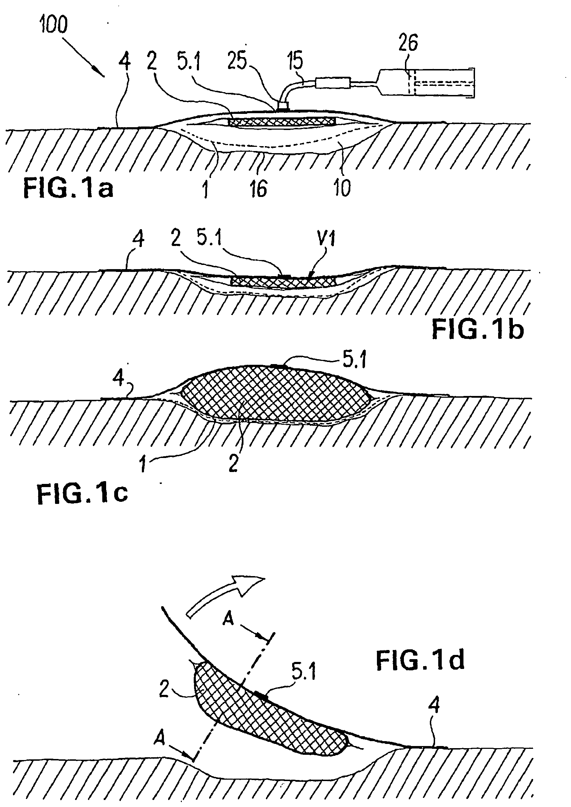

In den Figuren 1a bis 1d, 2, 3 und 8 ist eine Vorrichtung 100 zur Wundbehandlung dargestellt, bestehend aus einem folienartigen Wundabdeckungselement 4, einem schleimhautfreundlichen Folienelement 1 und einem dazwischen liegenden Absorptionskörper 2. Die Vorrichtung ist in Draufsicht auf ihre Flachseite etwa rechteckig und weist abgerundete Ecken 3 auf. An wenigstens einer Ecke des Wundabdeckungselementes 4 ist eine Abziehzunge 12 eingebracht. Außerdem ist eine sterile, luftdichte Verpackung (nicht dargestellt) vorgesehen.

Das aus einer flüssigkeitsundurchlässigen, transparenten Folie bestehende Wundabdeckungselement 4 ist relativ steif, d. h. es schrumpft nicht im nicht benutzten und im am Körper des Patienten angelegten Zustand. Das Wundabdeckungselement 4 ist an seiner Peripherie 8 mit einer Klebefläche 6 zum Ankleben der Vorrichtung an die Haut des Patienten versehen.

Der Absorptionskörper 2 besteht aus einer Lage 22 eines vliesartigen, textilen Materials, das Cellulosefasern umfaßt und mit Superabsorberteilchen (Super-Absorbing-Polymers, SAP), im vorliegenden Fall mit Natriumacrylat-Acrylsäure-Polymerisat durchsetzt ist. Außerdem ist der Absorptionskörper 2 mit nanokristallinen, silberhaltigen Substanzen angereichert, die mikrobizid wirken. Die Cellulosefasern wirken als Zwischenspeicher der spontan beaufschlagten Flüssigkeitsmengen und als eine Art Transportmittel, mit dem die Wundsekrete bis zum Superabsorber hin gelangen.

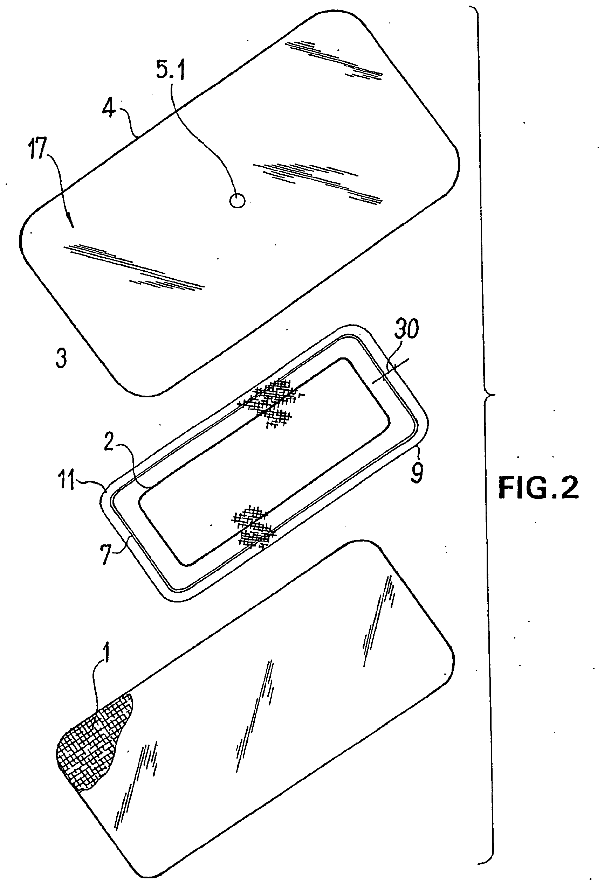

Die Lage 22 ist von einer flüssigkeitsdurchlässigen, ebenfalls textilen, mit einer umlaufenden Ultraschallnaht 7 verschweißten Hülle 11 umgeben. Wie insbesondere der Fig. 2 zu entnehmen ist, weist die Hülle 11 einen peripheren Überstand 30 an Hüllenmaterial auf, der zwischen der Ultraschallnaht 7 und einem äußersten Umfang 9 der Hülle 11 liegt. Die Aufgabe des Überstandes 30 ist, die Wunde vor der schmerzhaften Berührung mit der Naht zu verhindern.

Das der Wunde zugewandte Folienelement 1 ist aus einem flüssigkeitsdurchlässigen, hauchdünnen, schleimhautfreundlichen Material angefertigt. Das Folienelement 1 trägt auch dem Schutz vor Berührung mit der Ultraschallnaht 7 bei.

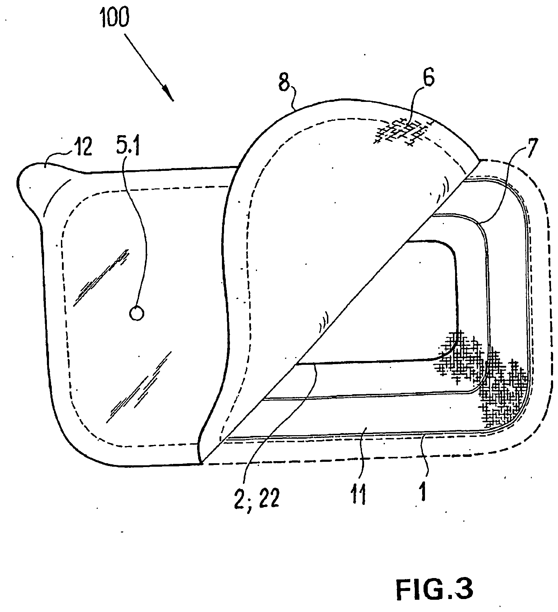

Ferner ist am Wundabdeckungselement 4 eine Anschlußstelle 5.1 zur Gasevakuierung und zur Kontrolle des Unterdrucks eingebracht. Gemäß den Figuren 1a bis 1d und Fig. 2 ist die Anschlußstelle 5.1 etwa mittig angeordnet. Sie kann jedoch an beliebiger Stelle am Wundabdeckungselement liegen, beispielsweise in der Nähe der Peripherie 8, wie in der Fig. 3 gezeigt worden ist.

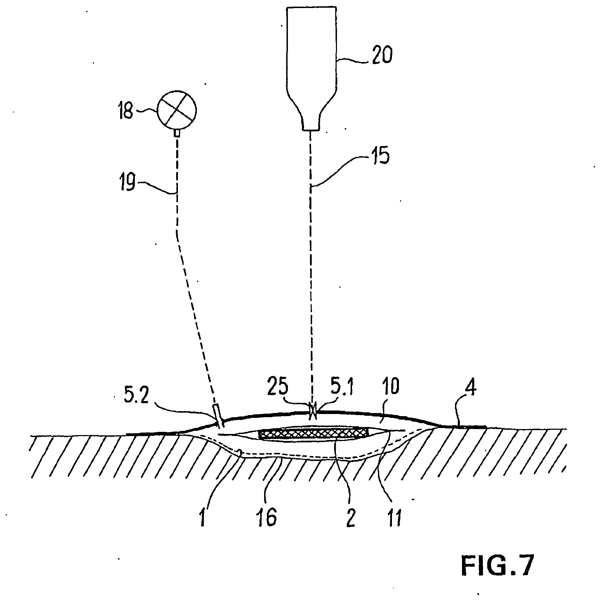

Die Vorrichtung gemäß der Fig. 7 weist zwei Anschlußstellen 5.1, 5.2 auf, von denen die eine, mittige zur Luftevakuierung und die zweite, seitliche zur Unterdruckkontrolle dient. An die mittige Anschlußstelle 5.1 ist über eine Schlauchleitung 15 eine Vakuumflasche 20, dagegen an die seitliche Anschlußstelle 5.2 ebenfalls über einen Verbindungsschlauch 19 ein Druckmanometer 18 angeschlossen ist. Die Beschreibung der Fig. 7 bezieht sich selbstverständlich auf die an die Haut des Patienten angeklebte Vorrichtung.

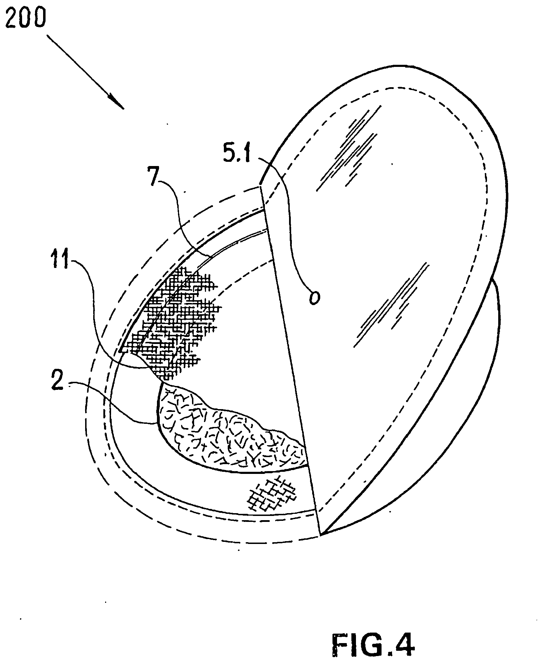

Die Fig. 4 zeigt eine Vorrichtung 200, die sich von der in der Fig. 1 dargestellten Vorrichtung 100 lediglich durch ihre runde bzw. ovale Außenkontur unterscheidet.

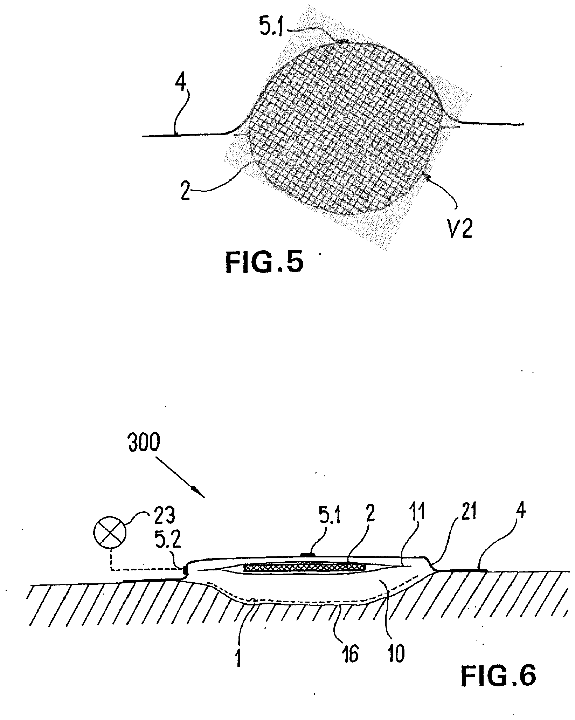

Eine andere Ausführungsform (Bezugszahl 300) der Vorrichtung zeigt schematisch die Fig. 6. Die Vorrichtung 300 weist ein schalenförmiges Wundabdeckungselement 4 auf, das es ermöglicht, die Anschlußstelle 5.2 an seinem seitlichen Mantel 21 einzubringen. An die Anschlußstelle 5.2 kann nach Bedarf eine Vakuumpumpe 23, gegebenenfalls mit Druckregulierung, angeschlossen sein.

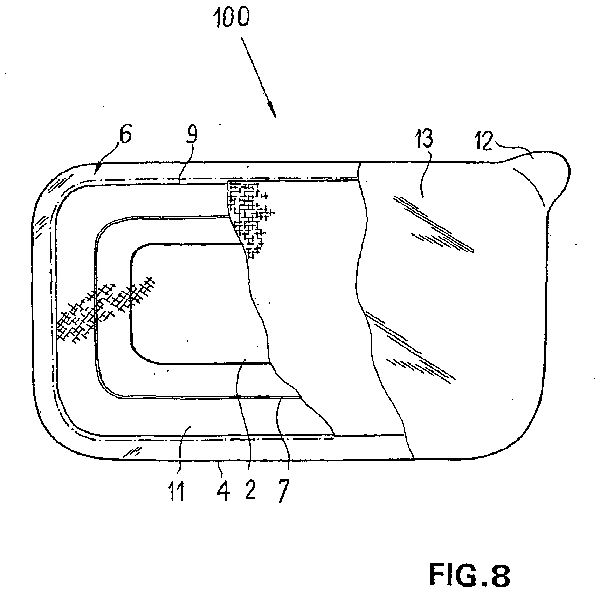

Ein an die Klebefläche 6 des Wundabdeckungselementes angeklebtes, silikonbeschichtetes Abziehfolienelement 13 (vgl. Fig. 8) hält die Teile der Vorrichtung 100; 200 oder 300 zusammen und schützt vor äußeren Einflüssen. Flächenmäßig ist das Abziehfolienelement 13 dem Wundabdeckungselement 4 gleich.

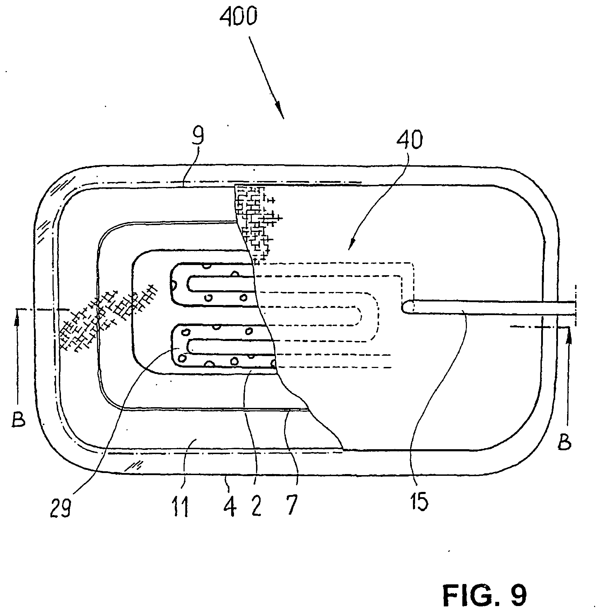

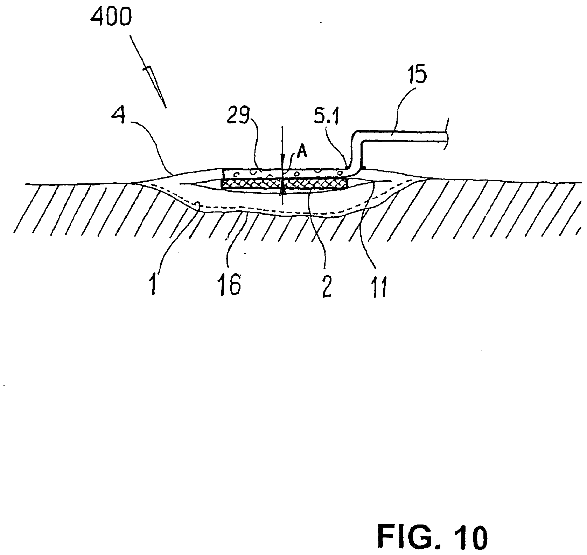

Wie die Figuren 9 und 10 zeigen, ist das Saugrohr 15 mit seinem Endstück 29 über die Anschlussstelle 5.1 geführt und zwischen dem folienartigen Wundabdeckungselement 4 und der Hülle 11 mäanderartig verlaufend platziert. Das Endstück 29 behält seine angegebene Form durch Unterbringung in einem nicht dargestellten, entsprechend profilierten Formstück aus offenzelligem Schaumstoff. So wird eine Konfiguration geschaffen, bei der der Durchmesser des Endstücks einen erforderlichen Abstand A zwischen dem Wundabdeckungselement 4 und dem umhüllten Absorptionsköper 2 definiert. Das Endstück 29 ist, wie an sich bekannt, mit mehreren Öffnungen versehen, die mit den Poren des Formstücks kommunizieren.

### Funktion:

Eine tiefe Wunde 16 wird durch das Ankleben der Vorrichtung 100 (Einwegvorrichtung) gemäß der Fig. 1 auf die Haut des Patienten vollständig bedeckt. Vorher wurde das in der Fig. 8 gezeigte Abziehfolienelement 13 entfernt, das eine periphere Klebefläche 6 an der Unterseite des Wundabdeckungselementes 4 freigibt. Mit einer sterilisierten Pinzette (nicht dargestellt) wurde zuerst das schleimhautfreundliche Folienelement 1, dann der flache Absorptionskörper 2 samt Hülle 11 vorsichtig auf die Wundoberfläche gelegt und erst danach das Wundabdeckungselement 4 um die Wunde herum angeklebt. Durch das Ankleben der Vorrichtung auf die Haut ist ein Raum 10 zwischen dem Wundabdeckungselementes 4 und der Wundoberfläche entstanden. An die mittige, mit einem einfachen Einwegrückschlagventil 25 (vgl. Fig. 1a) mit Propfen versehene Anschlußstelle 5.1 wurde über die vorgenannte Schlauchleitung 15 eine ärztliche Injektionsspritze 26 angeschlossen. Da der Raum 10 abgedichtet ist, können mit Hilfe der Injektionsspritze die im Raum befindlichen Gase evakuiert werden. Den Zustand zeigt die Fig. 1b. Die flachen Elemente der Vorrichtung liegen auf der Wundoberfläche an. Der inzwischen mit Hilfe eines nicht dargestellten Vakuum-Indikators bemessene Unterdruck betrug etwa 100 ml Hg. Hierbei kann die zylindrische Mantelfläche der Injektionsspritze mit einer entsprechenden, experimental definierten Unterdruck-Skala versehen sein.

Die aus der Wunde heraustretenden Wundsekrete gelangen in den Absorptionskörper 2 und bewirken eine Kompression unterhalb des Wundabdeckungselementes 4. Nach dem Ansaugen von Wundsekreten nimmt das Volumen des Absorptionskörpers 2 stark zu (vgl. Fig. 1c). Da der Absorptionskörper 2 flächenmäßig etwa 40% kleiner als die Hülle 11 ist, nähert sich er einer Kreisform (vgl. Fig. 5) an.

Die verbrauchte Vorrichtung 100 wird jetzt durch das Anheben der Abziehzunge 12 und mit Hilfe der Pinzette vorsichtig aus dem Bereich der Wunde entfernt. Nach Bedarf kann auf die Wunde eine neue Einwegvorrichtung aufgeklebt werden.

Der Quellvorgang des an der Wunde eingesetzten Absorptionskörpers wirkt auf den Heilungsprozess vorteilhaft, da der Absorptionskörper über eine Zunahme seines Gewichts einer Hypergranulation des Wundgewebes über das Hautniveau derartig mechanisch entgegenwirkt, indem sich eine der Körpermitte des Patienten zugewandte Drucksituation ergibt, die durch die Fixation unter der angesogenen äußeren Folie addiert wird. Auf dieser Weise kann die Haut - nach dem Wundheilungsprozess - einfacher zusammengenäht werden, da sie von dem übrigen Hautniveau nicht absteht. Dies bringt auch gewisse kosmetische Vorteile mit sich.

### Bezugszeichenliste:

- 1: Folienelement
- 2: Absorptionskörper
- 3: Ecke
- 4: Wundabdeckungselement
- 5.1; 5.2: Anschlußstelle
- 6: Klebefläche
- 7: Ultraschallnaht
- 8: Peripherie
- 9: Umfang (v. 11)
- 10: Raum
- 11: Hülle
- 12: Abziehzunge
- 13: Abziehfolienelement
- 15: Schlauchleitung (Saugrohr)
- 16: Wunde
- 17: Außenseite (v. 4)
- 18: Druckmanometer
- 19: Verbindungsschlauch
- 20: Vakuumflasche
- 22: Lage
- 23: Vakuumpumpe

- 25: Ventil
- 26: Injektionspritze

- 29: Endstück
- 30: Überstand

- 40: Druckverteiler
- 100: Vorrichtung
- 200: Vorrichtung
- 300: Vorrichtung
- 400: Vorrichtung

- A: Abstand

## Patentansprüche

1. Vorrichtung (100; 200; 300; 400) zur Wundbehandlung des menschlichen oder tierischen Körpers unter Einsatz von Unterdruck, aufweisend:
- ein gasdichtes Wundabdeckungselement (4), das im am Körper des Patienten angelegten Zustand einen zwischen der jeweiligen Wunde und dem Wundabdeckungselement verbleibenden Wundraum (10) bildet,
- wenigstens eine Anschlussstelle (5.1; 5.2), die mit dem Wundraum (10) in Kontakt steht und über welche die im Wundraum (10) befindliche Luft evakuiert werden kann,
- wenigstens einen flächenhaften Absorptionskörper (2), der im Wundraum (10) unterhalb des Wundabdeckungselementes (4) anzuordnen ist,
**dadurch gekennzeichnet, dass**
- der Absorptionskörper (2) wenigstens eine in eine Hülle (11) eingefasste Lage (22) eines mit superabsorbierenden Partikeln durchsetzten Textilabschnittes (2) ist, wobei die Hülle (11) flüssigkeitsdurchlässig ist und Poren aufweist, deren Größe die der superabsorbierenden Partikeln im Wesentlichen nicht überschreitet,
- der in den Wundraum (10) einzulegende Absorptionskörper (2) ein Anfangsvolumen (V1) aufweist, das sich im Laufe des Absorptionsprozesses vergrößert und ein Endvolumen (V2) annimmt, so dass die absorbierten Wundsekrete - bedingt durch die Porengröße der Hülle (11) - innerhalb des Absorptionskörpers (2) und damit unterhalb des Wundabdeckungselementes bis zur Entfernung des Absorptionskörpers aus dem Wundraum verbleiben,
- die Lage (22) in Draufsicht auf ihre Flachseite eine Flächenausdehnung hat, die um 3% bis 90% kleiner ist als die der flachgelegten Hülle (11).

2. Vorrichtung nach Anspruch 1, **dadurch gekennzeichnet, dass** der Absorptionskörper (2) ganzflächig mit dem Wundabdeckungselement (4) unter Belassung einer freien Peripherie (8) am Wundabdeckungselement verklebt ist.

3. Vorrichtung nach Anspruch 1, **dadurch gekennzeichnet, dass** sich der Absorptionskörper (2) in der Nähe seiner gesamten Füllungskapazität - im Querschnitt des Absorptionskörpers gesehen - einer Kreisform angenähert ist.

4. Vorrichtung nach Anspruch 1, **dadurch gekennzeichnet, dass** der Textilabschnitt des Absorptionskörpers (2) aus Zellstoff-Vlies besteht.

5. Vorrichtung nach Anspruch 1, **dadurch gekennzeichnet, dass** diese ein flüssigkeitsdurchlässiges, schleimhautfreundliches Schutzelement (1) beinhaltet, das im am Körper des Patienten angelegten Zustand auf einer dem Wundabdeckungselement (4) gegenüberliegenden Seite des Absorptionskörpers (2) angeordnet ist und in Draufsicht eine Flächengröße hat, die etwa so groß ist wie die des umhüllten Absorptionskörpers (2).

6. Vorrichtung nach Anspruch 1, **dadurch gekennzeichnet, dass** das Wundabdeckungselement (4) im am Körper des Patienten angelegten Zustand nicht schrumpft.

7. Vorrichtung nach einem der Ansprüche 1 bis 6, **dadurch gekennzeichnet, dass** das Wundabdeckungselement (4) auf seiner Außenseite (17) bedruckbar ist.

8. Vorrichtung nach einem der vorhergehenden Ansprüche, **dadurch gekennzeichnet, dass** die Anschlussstelle (5.1; 5.2) ein Ventil (25) aufweist.

9. Vorrichtung nach einem der vorhergehenden Ansprüche, **dadurch gekennzeichnet, dass** an die Anschlussstelle (5.1; 5.2) eine Schlauchleitung (15; 19) anschließbar ist.

10. Vorrichtung nach einem der vorhergehenden Ansprüche, **dadurch gekennzeichnet, dass** an die Anschlussstelle (5.1; 5.2) direkt oder an die Schlauchleitung (15; 19) ein Druckmanometer (18) anschließbar ist.

11. Vorrichtung nach einem der vorhergehenden Ansprüche, **dadurch gekennzeichnet, dass** an die Schlauchleitung (15; 19) wenigstens eine Vakuumflasche (20) oder eine Vakuumpumpe (23) anschließbar ist.

12. Vorrichtung nach einem der vorhergehenden Ansprüche, **dadurch gekennzeichn t,** dass an die Anschlußstelle (5.1; 5.2) oder an die Schlauchleitung (15; 19) eine Kolben-Zylinder-Anordnung, wie ärztliche Spritze (26), anschließbar ist.

13. Vorrichtung nach einem der vorhergehenden Ansprüche, **dadurch gekennzeichnet, dass** in diese ein Druckindikator integriert ist.

14. Vorrichtung nach einem der vorhergehenden Ansprüche, **dadurch gekennzeichnet, dass** der Absorptionskörper (2) und/oder das flüssigkeitsdurchlässige Schutzelement (1) bei der am Körper des Patienten angelegten Vorrichtung auswechselbar ist.

15. Vorrichtung nach einem der vorhergehenden Ansprüche, **dadurch gekennzeichnet, dass** die Hülle (11) des Absorptionskörpers (2) durch Ultraschallnähte vernäht ist.

16. Vorrichtung nach einem der vorhergehenden Ansprüche, **dadurch gekennzeichnet, dass** dem Material des Absorptionskörpers (2) und/oder des Schutzelementes den Wundheilungsprozess beeinflussende (1) Wirksubstanzen, beispielsweise nanokristalline Silberpartikeln appliziert sind.

17. Vorrichtung nach einem der vorhergehenden Ansprüche, **dadurch gekennzeichnet, dass** der Absorptionskörper an seinem Umfang einen Überstand (30) an Hüllenmaterial aufweist.

18. Vorrichtung nach einem der vorhergehenden Ansprüche, **dadurch gekennzeichnet, dass** das Schutzelement (1) folienartig ist.

19. Vorrichtung nach einem der vorhergehenden Ansprüche, **dadurch gekennzeichnet, dass** das Schutzelement (1) ein textiler Materialabschnitt ist.

20. Vorrichtung nach einem der vorhergehenden Ansprüche, **dadurch gekennzeichnet, dass** das Schutzelement (1) aus Schaumstoff besteht.

21. Vorrichtung nach einem der vorhergehenden Ansprüche, **dadurch gekennzeichnet, dass** das Schutzelement (1) eine unterhalb des Absorptionskörpers (2) liegende, lose Schüttung aus Vlies- oder Schaumstoffstücken ist.

22. Vorrichtung nach einem der vorhergehenden Ansprüche, **dadurch gekennzeichnet, dass** unterhalb des Wundabdeckungselementes (4), jedoch oberhalb des umhüllten Absorptionskörpers (2) ein mit der Hülle (11) des Absorptionskörpers (2) kontaktierender Druckverteiler (40) angeordnet ist.

23. Vorrichtung nach Anspruch 22, **dadurch gekennzeichnet, dass** der Druckverteiler (40) ein Formstück aus gasdurchlässigem Schaumstoff ist.

24. Vorrichtung nach Anspruch 22, **dadurch gekennzeichnet, dass** der Druckverteiler (40) ein schlaufen- oder mäanderartig verlaufendes Endstück (29) des durch die Anschlussstelle (5.1, 5.2) geführten Saugrohrs (15) aufweist, welches Endstück (29) einen Abstand (A) zwischen dem Wundabdeckungselement (4) und dem umhüllten Absorptionskörper (2) definiert.

## Claims

1. Device (100; 200; 300; 400) for treating wounds of the human or animal body using a vacuum comprising
- a gas-tight wound-covering element which, when placed in contact with the body of the patient, forms a wound space which remains between the respective wound and the wound-covering element,
- at least one connecting site (5.1, 5.2), which is in contact with the wound space (10) and over which the air in the wound space can be evacuated,
- at least one two-dimensional absorption body (2), which is to be disposed in the wound space (10) underneath the wound covering element (4),
**characterized in that**
- the absorption body (2) is at least one layer (22) of a textile section (2), which is enclosed in an envelope (11) and interspersed with super-absorbing particles, the envelope (11) being liquid permeable and having pores, the size of which essentially does not exceed that of the super-absorbing particles,
- the absorption body (2), which is to be placed in the wound space (10), has an initial volume (V1), which enlarges in the course of the absorption process and assumes a final volume (V2), such that, due to the size of the pores of the envelope (11), the absorbed wound secretions remain within the absorption body (2) and, with that, below the wound covering element, until the absorption body is removed from the wound space,
- the layer (22), in a plan view of its flat side, has an areal extent, which is 3% to 90% smaller than that of the envelope (11), when the latter is placed flat.

2. The device of claim 1, wherein the absorption body (2) is glued over its whole surface to the wound-covering element (4), a periphery (8) at the wound-covering element being left free.

3. The device of claim 1, wherein the absorption body (2), when filled close to its capacity, approaches a circular shape in cross-section.

4. The device of claim 1, wherein the textile section of the absorption body (2) consists of a nonwoven cellulose material.

5. The device of claim 1, further comprising a liquid-permeable, mucous membrane-compatible protective element (1), which, when placed in contact with the body of the patient, is disposed on a side of the absorption body (2) opposite to the wound-covering element (4) and, in plan view, is approximately equal in area to the enveloped absorption body (2).

6. The device of claim 1, wherein the wound-covering element (4) does not shrink when in contact with the body of the patient.

7. The device of claim 1 to 6, wherein an external surface (17) of the wound-covering element (4) is capable of supporting an ink print.

8. The device according to at least one of the preceding claims, wherein the connecting site (5.1, 5.2) comprises a valve (25).

9. The device according to at least one of the preceding claims, wherein the connecting site (5.1, 5.2) is connectable to a hose line (15; 19).

10. The device according to at least one of the preceding claims, wherein a pressure manometer (18) is connectable directly to the connecting site (5.1, 5.2) or to the hose line (15; 19).

11. The device according to at least one of the preceding claims, wherein at least one vacuum bottle (20) or one vacuum pump (23) is connectable to the hose line (15; 19).

12. The device according to at least one of the preceding claims, wherein a piston and cylinder arrangement such as a medical syringe is connectable to the connecting site (5.1; 5.2) or to the hose line (15; 19).

13. The device according to at least one of the preceding claims, wherein a pressure indicator is integrated in the device.

14. The device according to at least one of the preceding claims, wherein the absorption body (2) and/or the liquid-permeable protective element (1) is exchangeable when the device is in contact with the body of the patient.

15. The device according to at least one of the preceding claims, wherein the envelope (11) of the absorption body (2) is sewn by ultrasonic seams.

16. The device according to at least one of the preceding claims, wherein active substances, such as nanocrystalline silver particles, which affect the wound-healing process, are applied to the material of the absorption body (2) and/or to the protective element.

17. The device of according to at least one of the preceding claims, wherein the absorption body, at its periphery, has an overhang (30) of enveloping material.

18. The device according to at least one of the preceding claims, wherein the protective element (1) is film-like.

19. The device according to at least one of the preceding claims, wherein the protective element (1) is a section of textile material.

20. The device according to at least one of the preceding claims, wherein the protective element (1) comprises a foam material.

21. The device according to at least one of the preceding claims, wherein the protective element (1) is a loose bed of nonwoven or foam pieces, which are underneath the absorption body (2).

22. The device according to at least one of the preceding claims, wherein a pressure distributor (40), contacting the envelope (11) of the absorption body (2), is disposed below the wound covering (4) element, yet above the enveloped absorption body (2).

23. The device according to claim 22, wherein the pressure distributor (40) is a template of a foam material, which is permeable to gases.

24. The device according to claim 22, wherein the pressure distributor (40) has a loop-like or meandering end piece (29) of the suction tube (15) passed through the connecting site (5.1; 5.2), which end piece (29) defines a distance (A) between the wound-covering element and the enveloped absorption body (2).

## Revendications

1. Dispositif (100 ; 200 ; 300 ; 400) pour le traitement de plaie du corps humain ou animal avec application d'une dépression, comportant :
- un élément de recouvrement de plaie (4), étanche au gaz, qui forme un espace de plaie (10) restant entre la plaie considérée et l'élément de recouvrement de plaie lorsqu'il est appliqué sur le corps du patient,
- au moins un point de raccordement (5.1 ; 5.2) qui est en contact avec l'espace de plaie (10) et par l'intermédiaire duquel l'air se trouvant dans l'espace de plaie (10) peut être évacué,
- au moins un corps absorbant plat (2) qui doit être placé dans l'espace de plaie (10) sous l'élément de recouvrement de plaie (4),
**caractérisé en ce que**
- le corps absorbant (2) est au moins une couche (22), insérée dans une enveloppe (11), d'un morceau de textile (2) chargé de particules superabsorbantes, l'enveloppe (11) étant perméable au liquide et comportant des pores dont la dimension ne dépasse globalement pas celle des particules superabsorbantes,
- le corps absorbant (2) à placer dans l'espace de plaie (10) a un volume initial (V1) qui augmente au cours du processus d'absorption et qui prend un volume final (V2) de telle sorte que les secrétions de plaie absorbées restent - à cause de la dimension des pores de l'enveloppe (11) - à l'intérieur du corps absorbant (2) et donc sous l'élément de recouvrement de plaie jusqu'à ce que le corps absorbant soit retiré de l'espace de plaie,
- la couche (22) a en vue de dessus, sur son côté plat, une superficie qui est de 3 % à 90 % inférieure à celle de l'enveloppe mise à plat (11).

2. Dispositif selon la revendication 1, **caractérisé en ce que** le corps absorbant (2) est collé par toute sa surface à l'élément de recouvrement de plaie (4) tout en laissant une périphérie libre (8) sur l'élément de recouvrement de plaie.

3. Dispositif selon la revendication 1, **caractérisé en ce que** le corps absorbant (2) a - vu en coupe transversale du corps absorbant - une forme proche de la forme circulaire lorsqu'il est pratiquement au maximum de sa capacité de remplissage.

4. Dispositif selon la revendication 1, **caractérisé en ce que** le morceau de textile du corps absorbant (2) est en non-tissé de cellulose.

5. Dispositif selon la revendication 1, **caractérisé en ce que** celui-ci contient un élément protecteur (1) qui est perméable au liquide et toléré par les muqueuses, qui est placé sur celui des côtés du corps absorbant (2) qui est situé à l'opposé de l'élément de recouvrement de plaie (4) lorsqu'il est appliqué sur le corps du patient et qui a une surface en vue de dessus sensiblement aussi grande que celle du corps absorbant (2) enveloppé.

6. Dispositif selon la revendication 1, **caractérisé en ce que** l'élément de recouvrement de plaie (4) ne se rétracte pas lorsqu'il est appliqué sur le corps du patient.

7. Dispositif selon l'une des revendications 1 à 6, **caractérisé en ce que** l'élément de recouvrement de plaie (4) peut être soumis à une pression sur son côté extérieur (17).

8. Dispositif selon l'une des revendications précédentes, **caractérisé en ce que** le point de raccordement (5.1 ; 5.2) comporte une valve (25).

9. Dispositif selon l'une des revendications précédentes, **caractérisé en ce qu'**un tuyau souple (15 ; 19) peut être raccordé au point de raccordement (5.1 ; 5.2).

10. Dispositif selon l'une des revendications précédentes, **caractérisé en ce qu'**un manomètre (18) peut être raccordé directement au point de raccordement (5.1 ; 5.2) ou au tuyau souple (15 ; 19).

11. Dispositif selon l'une des revendications précédentes, **caractérisé en ce qu'**au moins un flacon à vide (20) ou une pompe à vide (23) peut être raccordé au tuyau souple (15 ; 19).

12. Dispositif selon l'une des revendications précédentes, **caractérisé en ce qu'**un dispositif à piston et cylindre, tel qu'une seringue à usage médical (26), peut être raccordé au point de raccordement (5.1 ; 5.2) ou au tuyau souple (15; 19).

13. Dispositif selon l'une des revendications précédentes, **caractérisé en ce qu'**un indicateur de pression est intégré dans celui-ci.

14. Dispositif selon l'une des revendications précédentes, **caractérisé en ce que** le corps absorbant (2) et/ou l'élément protecteur (1) perméable au liquide peuvent être changés lorsque le dispositif est appliqué sur le corps du patient.

15. Dispositif selon l'une des revendications précédentes, **caractérisé en ce que** l'enveloppe (11) du corps absorbant (2) est soudée par ultrason.

16. Dispositif selon l'une des revendications précédentes, **caractérisé en ce que** des substances actives influençant le processus de cicatrisation, par exemple des particules nanocristallines d'argent, sont appliquées sur le matériau du corps absorbant (2) et/ou de l'élément protecteur (1).

17. Dispositif selon l'une des revendications précédentes, **caractérisé en ce que** le corps absorbant comporte sur sa périphérie un débordement (30) de matériau d'enveloppe.

18. Dispositif selon l'une des revendications précédentes, **caractérisé en ce que** l'élément protecteur (1) est du genre feuille.

19. Dispositif selon l'une des revendications précédentes, **caractérisé en ce que** l'élément protecteur (1) est un morceau de matériau textile.

20. Dispositif selon l'une des revendications précédentes, **caractérisé en ce que** l'élément protecteur (1) est en un matériau alvéolaire.

21. Dispositif selon l'une des revendications précédentes, **caractérisé en ce que** l'élément protecteur (1) est constitué par des morceaux de non-tissé ou de matériau alvéolaire dispersés sous le corps absorbant (2).

22. Dispositif selon l'une des revendications précédentes, **caractérisé en ce qu'un** répartiteur de pression (40) en contact avec l'enveloppe (11) du corps absorbant (2) est placé en dessous de l'élément de recouvrement de plaie (4) mais au-dessus du corps absorbant (2) enveloppé.

23. Dispositif selon la revendication 22, **caractérisé en ce que** le répartiteur de pression (40) est une pièce façonnée en un matériau alvéolaire perméable au gaz.

24. Dispositif selon la revendication 22, **caractérisé en ce que** le répartiteur de pression (40) comporte un tronçon terminal (29) en forme de boucle ou de méandre du tuyau d'aspiration (15) passant par le point de raccordement (5.1, 5.2), lequel tronçon terminal (29) définit un écartement (A) entre l'élément de recouvrement de plaie (4) et le corps absorbant (2) enveloppé.
